(19) Europäisches Patentamt
European Patent Office
Office européen des brevets

(11) EP 3 633 044 A1

(12) EUROPEAN PATENT APPLICATION

(43) Date of publication:
**08.04.2020 Bulletin 2020/15**

(51) Int Cl.:
***C12Q 1/00*** *(2006.01)* ***G01N 27/327*** *(2006.01)*
***G01N 27/40*** *(2006.01)* ***G01N 33/483*** *(2006.01)*

(21) Application number: **18382700.5**

(22) Date of filing: **02.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitat Rovira I Virgili**
**43003 Tarragona (ES)**

(72) Inventors:
- **ANDRADE, Fancisco Javier**
  **43007 Tarragona (ES)**
- **BLONDEAU, Pascal**
  **43007 Tarragona (ES)**
- **Cánovas Martinez, Rocío**
  **43007 Tarragona (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

# (54) POTENTIOMETRIC SENSORS FOR THE SELECTIVE AND DIRECT DETECTION OF HYDROGEN PEROXIDE

(57) The present invention refers to a hydrogen peroxide selective electrode which comprises a redox sensitive surface consisting of platinum or gold and at least one layer of a proton exchange membrane on said redox sensitive surface, wherein said layer of a proton exchange membrane comprises
- A copolymer of Formula (II),

(II)

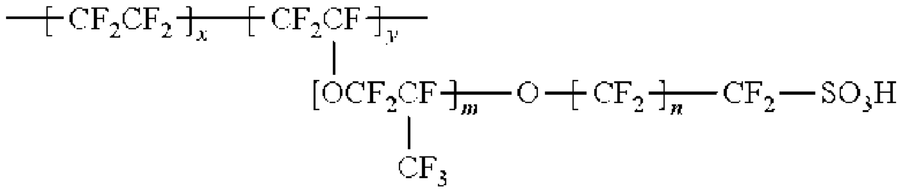

wherein x, y, m and n represent the numbers of repeat units, wherein m=0, n=1 and wherein the number of repeat units x and y are such that there are less than 15× units for each y.

EP 3 633 044 A1

## Description

### Field of the invention

The present invention pertains to the medical field, particularly to the detection of hydrogen peroxide in clinical samples by using potentiometric sensors. More particularly, the present invention solves the problem of providing devices for measuring hydrogen peroxide, preferably in a single undiluted whole blood drop out of the clinical laboratory.

### BACKGROUND OF THE INVENTION

The detection of hydrogen peroxide is always at the spotlight of chemical analysis. As an analyte, this substance plays a major role in many biological, industrial and environmental systems. Additionally, a plethora of bioanalytical assays and biosensors currently used in research as well as in routine clinical tests are based on the generation and detection of peroxide. To achieve high sensitivity and selectivity, conventional detection approaches usually rely on monitoring the byproduct of an enzymatic reaction of peroxide, often using horseradish peroxidase. Thus, developing alternative approaches that simplify the detection of peroxide, eliminating this last enzymatic reaction step, may have a significant impact in research and applications of chemical and biochemical analysis.

Open circuit potential (OCP) measurements are well known for their robustness, simplicity of instrumentation and operation, low power consumption and possibilities of miniaturization. However, very few practical options for the detection of peroxide have been reported. As a result, little progress in the development of potentiometric biosensing has been achieved. The use of a platinum (Pt) electrode for the potentiometric detection of hydrogen peroxide (e.g., US4340448A) was proposed several decades ago, but this approach shows poor sensitivity and a highly unspecific response. In fact, Pt electrodes are commonly used as generic, non-selective "redox probes". In a recent work, however, it has been reported a new and simple way to overcome many of these limitations by coating the Pt electrode with a Nafion membrane. Predictably, Nafion acts as a permselective barrier, preventing the interference caused by redox-active anions. More remarkable, though, a significant enhancement of the sensitivity, a stabilization of the potentiometric signal and an improvement on the reproducibility between electrodes was also found. While bare Pt electrodes show unstable responses with sensitivities for peroxide in the order of -30 mV/decade, Nafion-coated electrodes showed stable signals with sensitivities in the order of -120 mV/decade of peroxide. This sensitive and selective direct detection of hydrogen peroxide shows promise as a platform to build simple and robust potentiometric biosensors, as it was demonstrated through the development of paper-based enzymatic glucose sensors.

Beyond these practical applications, the mechanism of generation of the potentiometric response in this type of systems is a unique aspect that has not yet been properly discussed. Equilibrium-based arguments fail to explain the experimental evidence, such as the enhanced sensitivity obtained with the Nafion coating. As a matter of fact, it is well known that in the absence of a well-defined redox couple, the OCP of many metal electrodes -such as platinum- is not determined by a redox equilibrium, but is set by a mixed potential (MP) mechanism. Unlike equilibrium, where the electrode potential results from balancing the same forward and backwards reaction, MP are generated when different reactions take place on the surface of the electrode. These reactions may involve the electrode material, the solvent and other solution components. Oxidation reactions generate electrons, which are scavenged through reduction reactions. This Faradaic production of species creates anodic and cathodic exchange currents, which need to be balanced to avoid accumulation of charges. When this happens, the electrode reaches a stable non-equilibrium potential, usually called the mixed potential (MP), since it results from the balance of the driving force of different electrochemical reactions, as illustrated in Figure 1.

The theory of MP has become the basis for the study of corrosion phenomena and is also widely applied in other fields, such as heterogeneous catalysis. In chemical analysis, MP is extensively used for developing gas sensors, but few applications for sensing in solutions have been proposed. PB Hahn, DC Johnson, MA Wechter, AF Voigt Anal Chem 1974, 46, 553, explained the high sensitivity to oxygen of some sodium tungsten bronzes in terms of a MP mechanism. RK Meruva, ME Meyerhoff Anal Chem 1996, 68, 2022 studied the MP of some metal electrodes, such as Cu, Pt and Co and their potentiometric response to dissolved oxygen. They proposed the development of an oxygen sensor and, more interestingly, they demonstrated the development of a phosphate sensor based on the effect of this anion on the exchange current. Similarly, the MP of Zn electrodes and their use as potentiometric oxygen sensors has been proposed (C Vallieres, J Gray, S poncin, M Matlosz Electroanalysis 1998, 10, 191). Very recently, SJ Percival, AJ Bard, Anal Chem 2017, 89, 9843 proposed a very innovative approach to detect nanoparticles in solution using the MP of a Au electrode. They stressed the high sensitivity that can be obtained when monitoring the MP of a Pt microelectrode and showed that the manipulation of the kinetic parameters by the modification of the electrode geometry or surface can be used to tune the response of the system.

In summary, the use of MP-based sensors may offer a versatile route for the development of novel, highly sensitive and simple potentiometric detection schemes (Bard et al). Nevertheless, the control of all the factors that affect

the OCP in solution remain as a major challenge. Due to this different and kinetically controlled anodic and cathodic processes, one of the main characteristic of MP is that -even under zero net current- there is a continuous consumption and generation of chemical species at the electrode surface. Thus, the electrode MP does not show a Nernstian behavior and is highly sensitive to different conditions of the media, such as stirring, solution composition, etc. Thus, despite of the many advantages that it may bring, the difficulty controlling all these factors in solution is perhaps one of the most serious limitations for a broader applicability of MP in chemical analysis.

## BRIEF DESCRIPTION OF THE FIGURES

**Figure 1.** Representation of the processes in the electrode and the corresponding voltage and current plots.

**Figure 2.** Schematic representation of the manufacturing of WE (with different polymers such as Nafion or Aquivion), RE and the potentiometric sampling cell used during the measurements with real samples.

**Figure 3.** Time trace and calibration curve comparing the response to glucose of electrodes built with different polyelectrolytes Aquivion 12.5% "AQ|GOx|AQ" (blue hexagons) and Nafion 5% "Naf|GOx|Naf" (purple dots). All the measurements (N = 3) were performed in 0.1 M PBS (pH 7.4) at 25ºC.

**Figure 4.** Comparison of glucose determination (mM) in real blood samples obtained by the potentiometric biosensor (paper-based WE and paper-based RE) and glucometer device at 25ºC.

**Figure 5.** Cyclic voltagrams with Pt electrodes.

## DESCRIPTION OF THE INVENTION

Materials: For the examples described herein, two PFSA (Perfluorosulfonic acid) ionomers in the acid form were used: a long side chain PFSA; Nafion™ NR-40 with an Equivalent Weight EW=1000 g/eq, a short side chain PFSA Aquivion™ with EW=830 g/eq.

Perfluorosulfonic acid (PFSA) ionomers are polymers having a chemical moiety of the Formula (I):

(I)

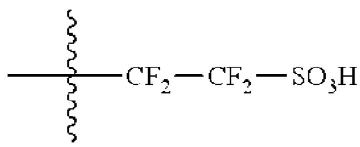

A preferred class of perfluorosulfonic acid ionomers are PFSA-polytetrafluoroethylene copolymers of Formula (II),

(II)

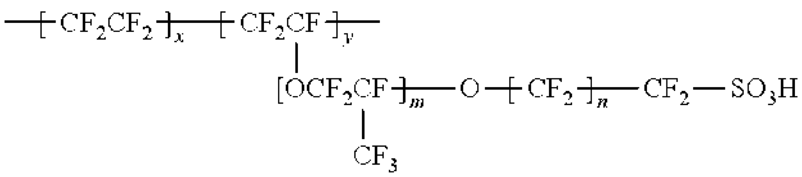

where x, y, m and n represent the numbers of repeat units. x and y are the numbers for tetrafluoroethylene and perfluorosulfonic acid repeat units respectively and m and n are the repeat units in the side chains of perfluorosulfonic acid blocks. x and y are equivalent weight dependent. For Nafion™ ionomer, a PFSA known in the art, the relationship between equivalent weight (EW) and m is EW=100x+446 so that the side chains are separated by around 14 $CF_2$ units in an ionomer with EW=1100. Preferably, the number of repeat units x and y are such that there are less than 15× units for each y and the value of m and n are integers between 0 and 5. Examples of PFSA copolymers known in the art are Nafion™: m=1 and n=1, Flemion™: m=0 or 1 and n=1 to 5, Aciplex™: m=0 or 3 and n=2 to 5, 3M™ ionomer: m=0 and n=2, Aquivion™: m=0 and n=1.

In the present invention, the MP of the Pt electrode is used to explain the potentiometric response to hydrogen peroxide, and the use of specific polyelectrolyte coatings is herein presented as a way to control the kinetic processes that lead to the resting potential of the electrode. It is well known that -like with many other metals- the resting potential of a Pt electrode in an electrolyte solution is not under equilibrium, but under a mixed potential (MP) regime, where oxidation (electron-generating) and reduction (electron-scavenging) reactions create anodic ($i_a$) and cathodic ($i_c$) exchange currents. Each one of these currents can be described by their Butler-Volmer equation:

$$i_k = i_k^o\left(e^{\frac{\alpha_k n_k F \eta_k}{RT}} - e^{\frac{(1-\alpha_k) n_k F \eta_k}{RT}}\right)$$

where the subscript "*k*" identifies a given reaction, $i_k$ is the total current, $i_k^o$ is the exchange current density, $\alpha_k$ is the symmetry factor, $n_k$ is the number of electrons exchanged in the rate-limiting step. The driving force of the reaction -the overpotential ($\eta_k$)- is the difference between the resting potential of the electrode (*E*) and the equilibrium potential of the reaction $(E_k^o)$ under study, and *F, R* and *T* are the Faraday's constant, universal gas constant and the absolute temperature, respectively. For large enough overpotential one of the directions of the reaction becomes negligible, i.e., the cathodic component of the anodic exchange current and the anodic component of the cathodic exchange current can be ignored. Therefore, the above equation can be then reduced to the Tafel approximation. Assuming that only one reaction contributes to each process, exchange currents can be expressed as:

$$i_a = i_a^o e^{\frac{\alpha_a n_a F \eta_a}{RT}}$$

$$i_c = i_c^o e^{\frac{(1-\alpha_c) n_c F \eta_c}{RT}}$$

When the value of potential is evaluated for the condition $i_a^o + i_c^o = 0,$ the MP of the system can obtained. Then, under a MP regime there is a net non-zero value of overpotential ($\eta$) that reaches a steady state due to a zero-value of the total current. Because of this, the OCP of these system does not show a Nernstian dependence, but it is controlled by the Tafel relationships that describe the exchange currents. Tafel equations are often expressed in the linear form:

$$E_a = K_a + \beta_a \log(i_a),\ \beta_a = 2.303\frac{RT}{\alpha_a n_a F}$$

$$E_c = K_c + \beta_c \log(i_c), \beta_c = 2.303\frac{RT}{(1-\alpha)_c n_c F}$$

where $E_a$ and $E_c$ are the electrode potential calculated for the anodic or cathodic exchange currents, $K_a$ and $K_c$ include a series of parameters and constant terms for each reaction and $\beta_a$ and $\beta_c$ are the Tafel slopes for the anodic and cathodic processes. Typically, for $\alpha$=0.5, expected Tafel slopes are 120 and 60 mV for one-electron and 2-electron transfer, respectively. However, additional factors that lead to more complex reaction mechanisms require corrections to the Tafel equation that result in a wider range of slopes.

Tafel slopes are key to understand the potentiometric response of these MP systems, since the OCP is linked to changes of the exchange currents. Any perturbation that leads to the readjustment of the exchange currents will result in a change of the electrode potential according to their Tafel plots. This is the reason why the OCP of many metal electrodes - including Pt- respond to the dissolved oxygen concentration. The oxygen reduction reaction (ORR):

$O_2 + 4H^+ + 4e^- \rightarrow 2H_2O$ ($E°$= *1.23 V*)

is one of the main contributors to the $i_c$ in the MP of many metals. Thus, changes on the concentration of $O_2$ modify $i_c$, forcing the system to reach a new MP. The sensitivities for dissolved $O_2$ -120 mV/decade for some bronzes, 40mV/decade for Co, and it 56 to 80 mV/decade for Cu- reflect the different kinetic conditions of this reaction, that yield different Tafel slopes. While the use of these metals as dissolved oxygen sensors has been proposed, a strict control over other factors affecting the exchange current -such as blocking the active sites on the electrode, modifications of the surface, adsorptions, etc is required. For example, a potentiometric phosphate sensor based on the modifications of the exchange currents produced by phosphate anions on a cobalt oxide surface has been reported. This is one of the major powers -and also the major weakness- of these mixed potential systems. From one side, they can offer great versatility, and potentially great sensitivity. From the other, they show strong dependence on several environmental factors that must be carefully controlled to be analytically useful.

In the case of Pt electrodes, the 2 major factors to consider when analyzing the MP are the cathodic and anodic exchange current, from one side, and the surface condition, from the other. Regarding the cathodic process, the strong dependence of the OCP with the concentration of dissolved $O_2$ evidences the major contribution of the ORR to the $i_c$. As a reduction reaction, the ORR acts removing electrons. Thus, a decrease of the ORR rate is reflected as drop of the electrode potential. Conversely, increasing the rate of this reaction drives the potential to higher values. The anodic reaction, on the other hand, is usually associated with the oxidation of Pt on the surface of the electrode. It has been reported that this reaction has a very low Tafel slope, which in practical terms means that the OCP of the Pt electrode will be mostly controlled by the cathodic process. Thus, Tafel slopes of the ORR will play a key role determining the sensitivity of the OCP. What is more relevant about the anodic process, though, is that it generates a layer of PtO, which inhibits the ORR, as it will be discussed below.

The ORR on Pt has received significant attention during the last decades because it is the limiting factor in the improvement of the performance of fuel cells (I Katsounaros, WB Schneider, JC Meier, U Benedikt, PU Biedermann, AA Auer, KJJ Mayrhofer, Phys. Chem. Chem. Phys., 2012, 14, 7384). The actual mechanism of the reaction is not fully elucidated, and the evidence suggest that different reaction pathways may occur depending on the conditions. The ORR on Pt is sluggish and does not proceed in a reversible way. As a multi-electron reaction, the ORR includes a number of elementary steps with different intermediaries (NM markovic, PN Ross Jr, Surface Science Reports 2002, 45, 117). Oxygen may be directly reduced to water (the 4-electron pathway) or be first reduced to hydrogen peroxide, which in turn may undergo different reactions. The most recent models and experimental evidence suggest that the mechanism of the ORR involves a first step of adsorption of $O_2$ onto Pt. Then a one-electron transfer is the rate-limiting step. Under these conditions, a Tafel slope of 120mV/decades (Markovic et al.) can be expected. Nevertheless, a number of factors may affect the reaction kinetics. Therefore, Tafel slopes of 60 and 120 mV/decade are typically reported, but values ranging from 40 to 80 mV/decade can be also found.

Since the first step of the reaction is the adsorption of $O_2$, the surface condition of the Pt electrode has a strong influence on the MP. Competitive adsorption of species on Pt has been studied by Markovic and Ross, who evaluated the ORR in the presence of sulphate and chloride anions. The effect of this spectator species on Pt has been also recently reviewed. In general, the adsorption of anions on the surface of the metal blocks the adsorption of oxygen, decreasing the ORR, and therefore affecting the MP. Evidently, from an analytical standpoint, this is a severe limitation for the use of bare Pt electrodes in solution, since the matrix will produce a significant and variable degree of interference.

Some of the most severe inhibitory effect on the ORR is produced by the presence of oxygenated species. The absorption of hydroxide groups, for example, blocks the active sites for the ORR. For this reason, a key factor controlling the electrochemical behavior of Pt is the surface oxide coverage. It has been long known that a clean Pt surface immersed in an aqueous solution is unstable, and it quickly oxidizes the first layers of metal until a stable potential is reached. Thus, Pt presents a potential-dependent surface oxide coverage, which has an inhibitory effect on the ORR, since $O_2$ requires clean Pt sites to adsorb. The generation of this oxide layer part of the mixed potential, since it is involved in the $i_a$. The anodic processes have traditionally received less attention, probably due to the lower kinetic impediments that it presents show. The metal oxidation:

$$2Pt + O_2 \rightarrow 2PtO \ (E° = 0.88)$$

is a fundamental part of the chemistry of Pt in aqueous solutions, as evidences in the cyclic voltagrams with Pt electrodes (figure 5).

Summarizing, the OCP of a Pt electrode is produced by a MP mechanism, where the ORR plays a fundamental role. Under ideal conditions, the kinetics of the ORR is controlled by the oxygen adsorption on free Pt sites available (and/or the change on the energy for adsorption of reaction intermediaries) followed by one electron transfer yield a Tafel slope of 120 mV. Nevertheless, surface effect will play an important role inhibiting the ORR. In particular, a potential-dependent surface oxide coverage will have a critical influence, since the total quantity and nature of the surface oxide species being reduced (degree of surface oxidation) has influence on the measured potential. Oxide-coverage factors have also been used to explain values of Tafel slopes below 120 mV/decades.

Interestingly, one of the oxygenated species that may block the ORR is the hydrogen peroxide. This molecule, which can be also found as intermediary in the ORR (depending on the reaction conditions) is weakly adsorbed in the surface on Pt, blocking free, active sites. Katsounaros has stressed the non-electrochemical nature of the interaction of peroxide with Pt. The OCP of a bare Pt electrode before and after the addition of peroxide produces a drop of the electrode potential. The drop of the electrode potential is the result of the blocking of the ORR, and not a redox reaction, as it has been previously proposed. While in principle this response could be used with analytical purposes, it is clear that the influence of the media would have a major effect on the response. Any increase on the concentration of anions will lead to a higher degree of adsorption of foreign species and a decrease on the response. Last, but not least, a characteristic of the MP is that there is a continuous generation and consumption of chemical species at the surface of the electrode, which make these systems highly dependent on the mass transport phenomena. **Any analytically useful approach should thus stabilize the concentration gradients on the surface of the electrode and minimize the interference effect of foreign species.**

In this regard, the use Nafion coatings on Pt electrodes was originally proposed several decades ago for increasing the performance of combustion cells. Since the solubility of oxygen in Nafion is almost 20 times higher than in water, it was believed that battery performance would increase likewise. The improvements obtained were not nearly as high as expected, but the use of Nafion was adopted and extended to other areas, such as amperometric sensing. In the potentiometric sensors, the use of Nafion shows three main advantages. First, it significantly increases the sensitivity to peroxide. Second, it prevents the interferences caused by negatively charged species. Third, it stabilizes the electrode response and electrode-to-electrode reproducibility. A Nafion-coated Pt electrode produces a higher resting potential of the coated electrodes, which can be ascribed to and enhancement of the ORR due to the chemical environment created by Nafion on the surface of the Pt. The exact nature of the Nafion-Pt interface is still a matter of study, but several general characteristics can be pinpointed. First, the perfluorosulphonate polymers are superacids, creating an environment with high proton activity on the surface of the metal. Second, the solubility of $O_2$ in Nafion is about 20 times higher than in water. Since the ORR is first order on the concentration of $O_2$ and $H^+$, and it depends on the availability of free Pt sites, the environment created by the Nafion coating produces a kinetic enhancement of the ORR, which leads to an increase on the electrode OCP. However, despite of the many advantages that Nafion coatings on Pt electrodes may bring, as illustrated in figure 4, such coatings only allow a linear range for the identification of molecules such as glucose between 0.3 mM and 3 mM, which constitutes a serious limitation for a broader applicability of MP in chemical analysis.

The authors of the present invention have thus tried using different coatings on Pt electrodes, different from Nafion, that produced a shift in the linear range obtained with Nafion and that at the same time significantly increased the sensitivity to peroxide, prevented the interferences caused by negatively charged species, and stabilized the electrode response and electrode-to-electrode reproducibility. In this sense, they use the following coating:

- copolymer of Formula (II) (from hereinafter referred to as "Aquivion"),

(II)

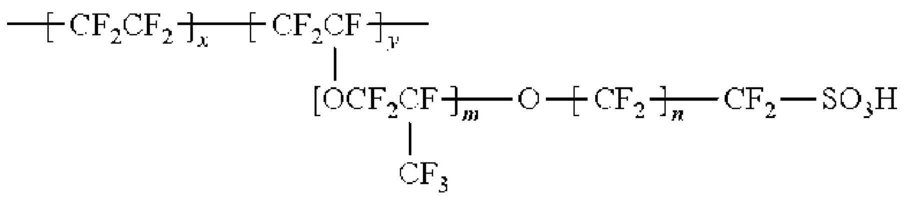

wherein x, y, m and n represent the numbers of repeat units, wherein m=0, n=1 and wherein the number of repeat units x and y are such that there are less than 15× units for each y

Said coating allowed a better control of the processes occurring at the electrode surface, providing an enhanced stability to the system. The potentiometric response observed was explained in terms of the blocking effect of the active sites of Pt by the weakly absorbed hydrogen peroxide, which leads to a drop of the electrode potential due to a decrease of the cathodic exchange current produced by the oxygen reduction reaction. Moreover, we herein demonstrate that the linear range of the detection of hydrogen peroxide can be tuned with the polymer coating. Noteworthy, the potentiometric response was shifted in terms of the linear range. In the case of using a Nafion coating, a linear range for the identification of glucose between 0.3 mM and 3 mM is obtained as illustrated in figure 4, (because of this, a dilution of blood samples is required to do monitor glucose levels). In contrast, the linear range obtained with Aquivion is shifted to higher concentrations, in particular between 3 mM and 10 mM, allowing a direct measurement of glucose in serum or blood. Consequently, a simplification during the analytical process of glucose detection within the clinical range might be carried out. Besides, new applications for these enzymatic sensors might be approached.

Furthermore, as the selectivity is also crucial in the characterization of the analytical performance of the electrodes, additions of the main interferents commonly found in blood were evaluated. During the studies of selectivity, **it was appreciated that Aquivion eliminates the redox interferences better than Nafion (see Table 1 with response to ascorbic acid).**

Therefore, a first aspect of the invention refers to a hydrogen peroxide selective electrode which comprises a redox sensitive surface consisting of platinum or gold and at least one layer of a proton exchange membrane on said redox sensitive surface, wherein said layer of a proton exchange membrane comprises

- A copolymer of Formula (II),

(II)

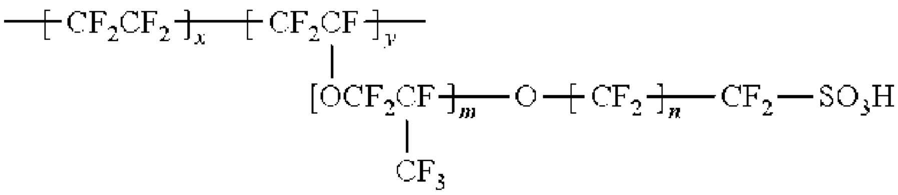

wherein x, y, m and n represent the numbers of repeat units, wherein m=0, n=1 and wherein the number of repeat units x and y are such that there are less than 15× units for each y.

In a preferred embodiment of the first aspect of the invention or of any of its preferred embodiments, said layer of a proton exchange membrane further comprises or conforms a mixture with a further PFSA copolymer selected from those of formula II wherein m=1 and n=1, or m=0 or 1 and n=1 to 5, or m=0 or 3 and n=2 to 5, or m=0 and n=2, and wherein x and y are such that there are less than 15× units for each y.

In yet another preferred embodiment of the first aspect of the invention or of any of its preferred embodiments, said layer of a proton exchange membrane further comprises a glucose oxidase entrapped therein or any oxidase with hydrogen peroxide as its product. On the other hand, a second aspect of the invention refers to a potentiometric cell capable of selectively measuring hydrogen peroxide comprising a selective electrode and a reference electrode, wherein the selective electrode comprises a redox sensitive surface consisting of platinum or gold and at least one layer of a proton exchange membrane on said redox sensitive surface as defined in the first aspect of the invention or in any of its preferred embodiments. Preferably, said hydrogen peroxide potentiometric cell comprises:

a. A reference electrode comprising a conductive material such as Ag/AgCl onto which a membrane comprising sodium chloride and PVB dissolved in an appropriate solvent such as methanol is deposited; and

b. A hydrogen peroxide selective electrode comprising a redox sensitive surface consisting of platinum or gold and at least one layer of a proton exchange membrane on said redox sensitive surface as defined in the first aspect of the invention or in any of its preferred embodiments.

It is noted that PVB or Butvar B-98 is understood as polyvinyl butyryl having a molecular weight between 40000-70000 g/mol with butyryl content between 78 and 80% weight per total weight of the polyvinyl butyryl (w/w), hydroxyl content between 18 and 20% (w/w) and acetate less than 2.5%, preferably between 1.5 and 2.5% (w/w).

In addition, a third aspect of the invention refers to the *in vitro* use of the hydrogen peroxide potentiometric cell of the second aspect of the invention or the hydrogen peroxide selective electrode of the first aspect of the invention or of any of its preferred embodiments, to selectively and directly determine the concentration of hydrogen peroxide in an aqueous solution. Preferably, said aqueous solution is a biological fluid such as whole blood, preferably undiluted whole blood, intracellular fluids, saliva, cerebrospinal fluid, blood sera, blood plasma, sweat and urine. More preferably, said aqueous solution is whole blood, in particular undiluted whole blood.

Furthermore, a fourth aspect of the invention refers to the hydrogen peroxide potentiometric cell of the second aspect of the invention, wherein said potentiometric cell preferably comprises a support made of paper which in turn comprises the selective electrode and the reference electrode, thus providing a paper-based sensor. It is however noted that other supports such as plastic, rubber, and textile can be used to practice the present invention. It is, of course, further noted that such paper-based sensors can be used to selectively and directly determine the concentration of hydrogen peroxide in an aqueous solution, wherein preferably said aqueous solution is a biological fluid such as whole blood, preferably undiluted whole blood, intracellular fluids, saliva blood sera and urine. In particular, said selective and direct determination of the concentration of hydrogen peroxide in an aqueous solution in turn determines the concentration of glucose, galactose, cholesterol, uric acid, lactic acid and amino acid in said solution.

The following examples merely illustrate the present invention and do not limit the same.

## EXAMPLES

## MATERIALS AND METHOD

### Reagents and materials

Whatman® qualitative filter paper, Grade 5, Glucose oxidase with 228.253 U/g (GOx), 30% (w/w) hydrogen peroxide ($H_2O_2$) standard solution, sodium hydrogen carbonate, sodium-L-ascorbate, sodium-lactate, urea, magnesium chloride, potassium hydrogen phosphate, glucose, fructose, uric acid and urea, Nafion® perfluorinated resin solution (5 wt% in a mixture of lower aliphatic alcohols and water, 45% water), Aquivion® D98-25BS consisting in a liquid dispersion, 25% in water, PFSA eq. wt. 980 g/mole $SO_3H$ with $CF_3$ polymer and chain ends as stabilizer was obtained in Sigma-Aldrich. All solutions were prepared using deionized water (18.2 $M\Omega \cdot cm^{-1}$ specific resistivity) obtained from Milli-Q PLUS (Millipore Corporation, Bedford, MA, USA).

Platinum sputtering was performed using a radiofrequency sputtering process (ATC Orion 8-HV, AJA International) operated at 3 mTorr, for 65s at 200W. Filter paper strips were placed inside the sputtering chamber to generate the electrodes. An adhesive plastic mask (0.3 mm thick) coated with an acrylic adhesive on one side (Arcare 8565, Adhesives Research Inc., Limerick, Ireland) was used to expose a given area of the coated paper and isolate the rest of the conductive surface.

### Instrumentation and electrochemical measurements

Electomotrive force (EMF) measurements were performed at room temperature (25°C) with a high input impedance ($10^{15}$ $\Omega$) EMF16 multichannel data acquisition device (Lawson Laboratories, Inc. Malvern). Laboratory measurements were made using a 4 mL cell in 0.1 M PBS (pH 7.4, 145 mM NACl) and artificial serum, briefly (140 mM NaCl, 29 mM $NaHCO_3$, 2.2 mM KCl, 0.8 MgCl, 1 mM Na-Lactate, 2.2 mM $K_2HPO_4$, 2.5 mM Urea) (pH 7.4) at 25ºC.

A commercial amperometric glucose sensor (Bayer® Contour glucometer) was used to compare glucose blood values determined by our paper-based potentiometric sensor.

### Fabrication of the glucose biosensor

The working electrode was built using Glucose oxidase entrapped between two layers of a polymeric matrix. Two different polymers (Nafion and Aquivion) were used The deposition of the polymeric matrices (either Nafion or Aquivion) was carried out by drop casting. Dilutions of Aquivion were performed with methanol ≥99.8%. For the reference electrode, a total of 9 μl of a reference membrane consisting of 78 mg PVB and 50 mg NaCl in 1 mL of methanol was drop-cast. To build the miniaturized cell, the enzymatic sensor was placed in between the sampling cell and connected to the measuring device through the conductive ends (Figure 2). This cell was kept at 4ºC when not in use. The sampling cell was designed and optimized for a sample volume of 25 μL.

## RESULTS

### Optimization and analytical performance comparison

The Figure 3 (time trace and calibration curve) exhibit the comparison of the potentiometric response to glucose obtained by enzymatic electrodes using either Nafion or Aquivion under the same conditions (PBS 0.1 M with pH of 7.4). Noteworthy, the potentiometric response is shifted in terms of the linear range. In the case of Nafion, a linear range between 0.3 mM and 3 mM is obtained, (because of this, a dilution of blood samples is required to do monitor glucose levels). In contrast, the linear range obtained with Aquivion is shifted to higher concentrations allowing a direct measurement of glucose in serum or blood. Consequently, a simplification during the analytical process of glucose detection within the clinical range could be carried out.

As the selectivity is also crucial in the characterization of the analytical performance of the electrodes, additions of the main interferents commonly found in blood were evaluated. During the studies of selectivity, Aquivion reduced the interference produced by ascorbic acid better than Nafion (Table 1).

**Table 1.** Comparison of analytical parameters using different configurations of the electrodes built with Nafion 5% and Aquivion 12.5% in 0.1 M PBS buffer (pH = 7.4). (Naf means nafion, GOx means glucose oxidase and AQ means Aquivion)

| | Naf\|GOx\|Naf | AQ\|GOx\|AQ |
|---|---|---|
| **Sensitivity (mV·decade$^{-1}$)** | -133.4 ± 11.7 | -133.2 ± 9.9 |
| **Linear range (mM)** | 0.3 - 3 | 1 - 30 |
| **Limit of detection (mM)** | 0.73 ± 0.001 | 0.30 ± 0.1 |
| **Time of response (sec)** | 80 | 95 |
| **Response to AA (mV)** | < 30 | < 10 |

*AA = Ascorbic Acid

**Real samples measurements**

All the previous experiments were performed in a 4 mL cell using a comercial double junction reference electrode. Hereinafter, the results shown in Figure 4 were carried out using the WE composed by the scheme Pt|AQ|GOx|AQand a paper-based RE (Figure 2). The main advantage, however, is that -due to the modified linear range- the Aquivion-based device does not require a prior dilution of the sample.

The validation of the potentiometric cell was carried out using 15 undilluted blood samples provided by different volunteers. The device was calibrated with three standards of glucose 1, 3.16 and 10 mM in artificial serum chosen as they are in the linear range of the sensor. Figure 4 displays the validation between values of glucose obtained using a comercial glucometer versus our potentiometric biosensor. This results represent a good linear correlation between both methods. The recoveries for the assays from blood samples are 95.6 ± 12.2%. This indicates that the blood matrix has no significant effect on glucose determination.

**Claims**

1. A hydrogen peroxide selective electrode which comprises a redox sensitive surface consisting of platinum or gold and at least one layer of a proton exchange membrane on said redox sensitive surface, wherein said layer of a proton exchange membrane comprises a copolymer of Formula (II),

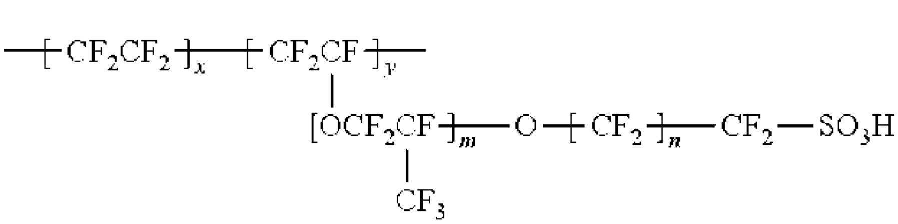

(II)

wherein x, y, m and n represent the numbers of repeat units, wherein m=0, n= 1 and wherein x and y are such that there are less than 15× units for each y.

2. The hydrogen peroxide selective electrode according to claim 1, wherein said layer of a proton exchange membrane further comprises a PFSA copolymer selected from those of formula II wherein m=1 and n=1, or m=0 or 1 and n=1 to 5, or m=0 or 3 and n=2 to 5, or m=0 and n=2 and wherein x and y are such that there are less than 15× units for each y.

3. The hydrogen peroxide selective electrode of any of claims 1 to 2, wherein said layer of a proton exchange membrane further comprises a glucose oxidase entrapped therein or any oxidase enzyme with hydrogen peroxidase as a product of the enzymatic reaction catalyse by said oxidase enzyme.

4. A potentiometric cell capable of selectively measuring hydrogen peroxide comprising a selective electrode and a reference electrode, wherein the selective electrode comprises a redox sensitive surface consisting of platinum or gold and at least one layer of a proton exchange membrane on said redox sensitive surface, wherein said membrane is as define in any of claims 1 to 3.

**5.** A potentiometric cell capable of selectively measuring hydrogen peroxide comprising:

a. A reference electrode comprising a support which in turn comprises a conductive material such as silver on to which a membrane comprising sodium chloride and PVB dissolved in an appropriate solvent such as methanol is deposited; and
b. A hydrogen peroxide selective electrode comprising a redox sensitive surface consisting of platinum or gold and at least one layer of a proton exchange membrane on said redox sensitive surface, wherein said membrane is as define in any of claims 1 to 3.

**6.** *In vitro* use of the potentiometric cell of any of claims 4 or 5, to selectively and directly determine the concentration of hydrogen peroxide in an aqueous solution.

**7.** The use according to claim 6, wherein said aqueous solution is a biological fluid such as whole blood, preferably undiluted whole blood, intracellular fluids, saliva, cerebrospinal fluid, blood sera, blood plasma, sweat and urine.

**8.** The use according to claim 6, wherein said aqueous solution is whole blood.

**9.** *In vitro* use of the hydrogen peroxide selective electrode of any of claims 1 to 3, to selectively and directly determine the concentration of hydrogen peroxide in an aqueous solution.

**10.** The potentiometric cell according to any of claims 4 or 5, wherein said potentiometric cell comprises a support which in turn comprises the selective electrode and the reference electrode, and wherein said support is made of paper, plastic, rubber, or textile.

**11.** The potentiometric cell according to claim 10, wherein the support is made of paper.

**12.** *In vitro* use of the paper-based sensor of claim 11, to selectively and directly determine the concentration of hydrogen peroxide in an aqueous solution. wherein preferably said aqueous solution is a biological fluid such as whole blood, preferably undiluted whole blood, intracellular fluids, saliva blood sera and urine.

**13.** The in vitro use according to claim 12, wherein said selective and direct determination of the concentration of hydrogen peroxide in an aqueous solution in turn determines the concentration of glucose in said solution.

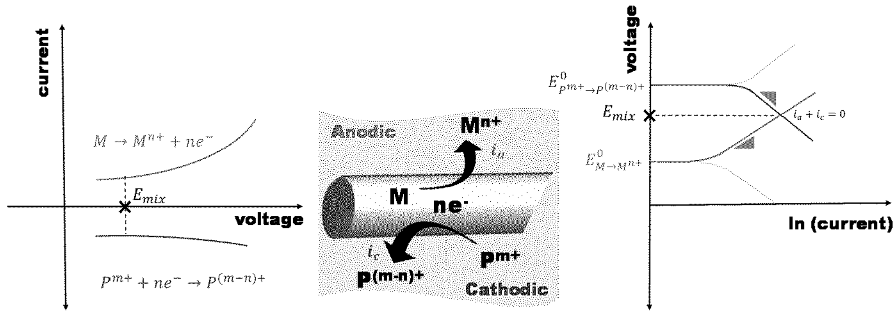


Figure 1

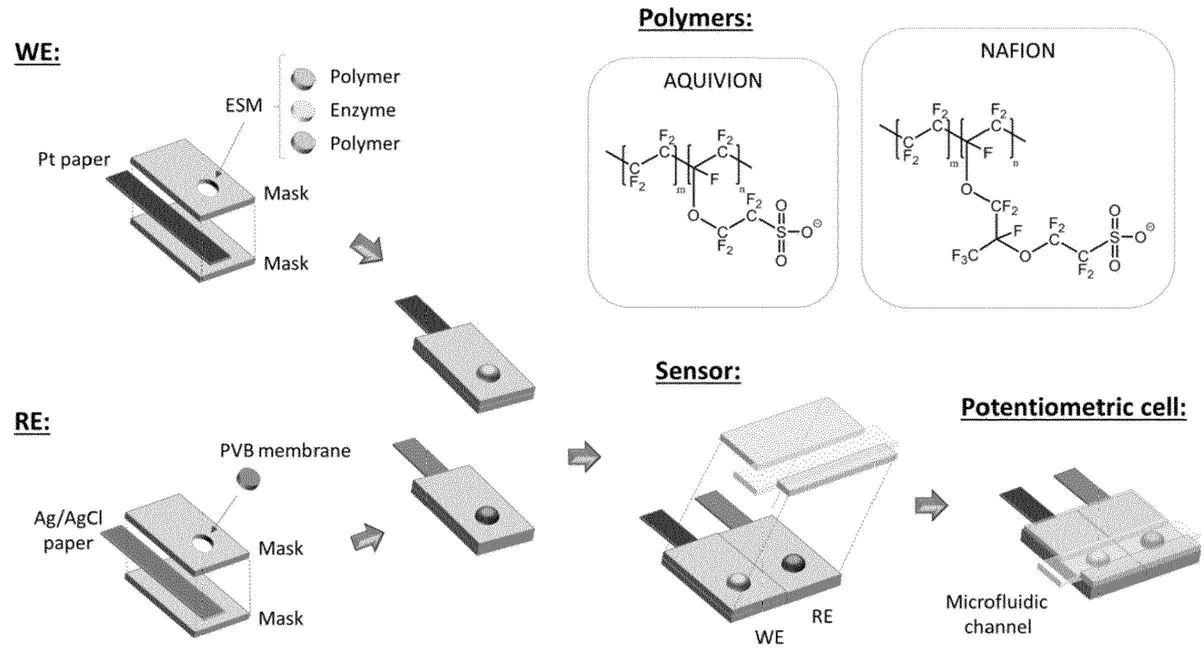


Figure 2

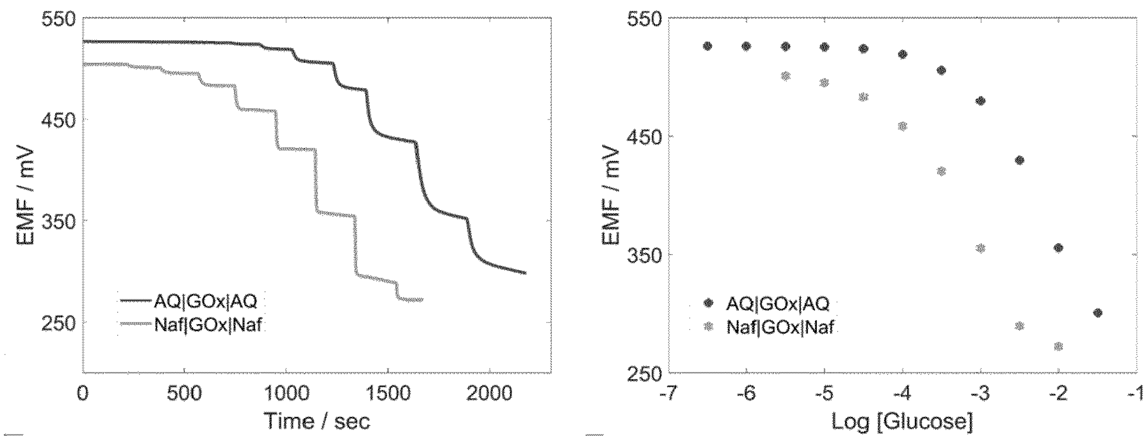


Figure 3

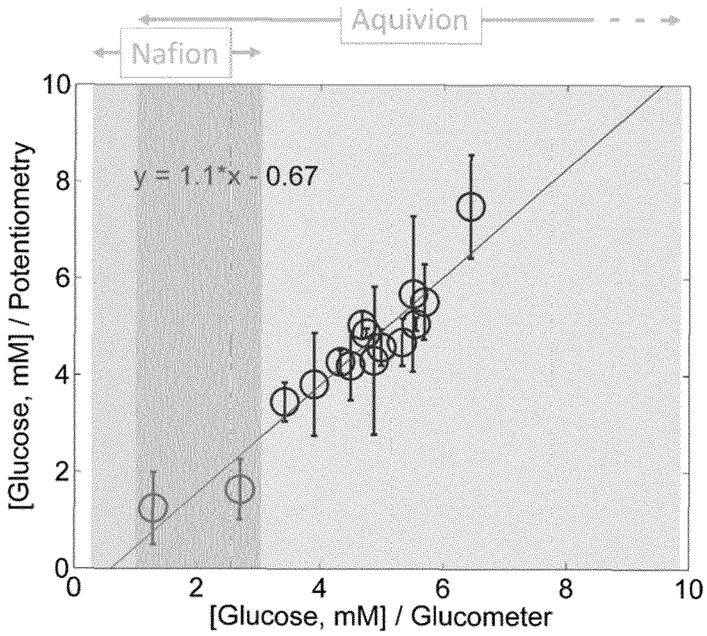


Figure 4

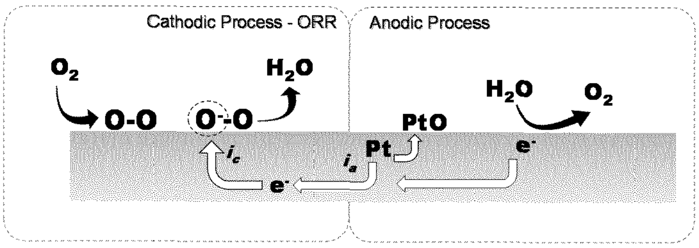


Figure 5

Europäisches Patentamt
European Patent Office
Office européen des brevets

# EUROPEAN SEARCH REPORT

Application Number
EP 18 38 2700

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | KR 2016 0083713 A (TAEMYUNG E&E COMPANY LTD [KR]) 12 July 2016 (2016-07-12)<br>* abstract;par. 127-135 * | 1<br>2-13 | INV.<br>C12Q1/00<br>G01N27/327<br>G01N27/40<br>G01N33/483 |
| X<br>Y | US 2015/144486 A1 (CHO CHULHO [KR] ET AL) 28 May 2015 (2015-05-28)<br>* abstract; reference example 4; par. 30 * | 2,3<br>1,4-13 | |
| Y | US 2015/273146 A1 (WARD WILLIAM KENNETH [US] ET AL) 1 October 2015 (2015-10-01)<br>* abstract; par. 36, 42, Fig. 1; par. 13 * | 1-13 | |
| Y | S. SIRACUSANO ET AL: "Performance analysis of short-side-chain Aquivion perfluorosulfonic acid polymer for proton exchange membrane water electrolysis", JOURNAL OF MEMBRANE SCIENCE, vol. 466, 1 September 2014 (2014-09-01), pages 1-7, XP055527746, NL ISSN: 0376-7388, DOI: 10.1016/j.memsci.2014.04.030<br>* section 4 * | 1-13 | TECHNICAL FIELDS SEARCHED (IPC)<br>C12Q<br>G01N |

The present search report has been drawn up for all claims

2

| Place of search | Date of completion of the search | Examiner |
|---|---|---|
| Munich | 28 November 2018 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 38 2700

This annex lists the patent family members relating to the patent documents cited in the above-mentioned European search report. The members are as contained in the European Patent Office EDP file on
The European Patent Office is in no way liable for these particulars which are merely given for the purpose of information.

28-11-2018

| Patent document cited in search report | Publication date | Patent family member(s) | Publication date |
|---|---|---|---|
| KR 20160083713 A | 12-07-2016 | NONE | |
| US 2015144486 A1 | 28-05-2015 | KR 20150061720 A | 05-06-2015 |
| | | US 2015144486 A1 | 28-05-2015 |
| US 2015273146 A1 | 01-10-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4340448 A


### Non-patent literature cited in the description


- **PB HAHN ; DC JOHNSON ; MA WECHTER ; AF VOIGT.** *Anal Chem,* 1974, vol. 46, 553
- **RK MERUVA ; ME MEYERHOFF.** *Anal Chem,* 1996, vol. 68, 2022
- **C VALLIERES ; J GRAY ; S PONCIN ; M MATLOSZ.** *Electroanalysis,* 1998, vol. 10, 191
- **SJ PERCIVAL ; AJ BARD.** *Anal Chem,* 2017, vol. 89, 9843
- **I KATSOUNAROS ; WB SCHNEIDER ; JC MEIER ; U BENEDIKT ; PU BIEDERMANN ; AA AUER ; KJJ MAYRHOFER.** *Phys. Chem. Chem. Phys.,* 2012, vol. 14, 7384
- **NM MARKOVIC ; PN ROSS JR.** *Surface Science Reports,* 2002, vol. 45, 117